# EUROPÄISCHE PATENTSCHRIFT

(11) **EP 1 186 669 B1**
(45) Veröffentlichungstag und Bekanntmachung des Hinweises auf die Patenterteilung: **25.01.2006**
(21) Anmeldenummer: 00119182.4
(22) Anmeldetag: 05.09.2000
(51) Int. Cl.: C12Q 1/68

(54) **Verfahren zur spezifischen Bestimmung von DNA-Sequenzen mittels paralleler Amplifikation**
Method for specific detection of DNS sequences using parallel amplification
Méthode de détection spécifique des séquences d'ADN utilisant l'amplification parallèle

(43) Veröffentlichungstag der Anmeldung: 13.03.2002
(73) Patentinhaber: Zeltz, Patrick, Dr., 79199 Kirchzarten (DE); Schneider, Stephan, 79102 Freiburg (DE)
(72) Erfinder: Zeltz, Patric, 79199 Kirchzarten (DE); Schneider, Stephan, 79102 Freiburg (DE)
(74) Vertreter: Schwabe - Sandmair - Marx

(56) Entgegenhaltungen:
- WO-A-90/11369
- WO-A-93/09250
- WO-A-93/21339
- WO-A-96/04404
- WO-A-96/26291
- WO-A-96/31622
- WO-A-97/32040
- WO-A-98/28438
- WO-A-99/05321
- HEAD ET AL: "NESTED GENETIC BIT ANALYSIS (N-GBA) FOR MUTATION DETECTION IN THE p53 TUMOR SUPPRESSOR GENE" NUCLEIC ACIDS RESEARCH,OXFORD UNIVERSITY PRESS, SURREY,GB, Bd. 25, Nr. 24, 1997, Seiten 5065-5071, XP002144794 ISSN: 0305-1048
- NEWTON: "PCR" 1997 , BIOS SCIENTIFIC PUBLISHERS , OXFORD XP002167652 3.7 Verschachtelte PCR * Seite 62 *
- CHENG J ET AL: "CHIP PRC II. INVESTIGATION OF DIFFERENT PCR AMPLIFICATION SYSTEMS IN MICROFABRICATED SILICON-GLASS CHIPS" NUCLEIC ACIDS RESEARCH,GB,OXFORD UNIVERSITY PRESS, SURREY, Bd. 24, Nr. 2, 1996, Seiten 380-385, XP000578332 ISSN: 0305-1048
- SHOFFNER M A ET AL: "CHIP PCR.I. SURFACE PASSIVATION OF MICROFABRICATED SILICON-GLASS CHIPS FOR PCR" NUCLEIC ACIDS RESEARCH,GB,OXFORD UNIVERSITY PRESS, SURREY, Bd. 24, Nr. 2, 1996, Seiten 375-379, XP000605272 ISSN: 0305-1048
- STRIZHKOV BORIS N ET AL: "PCR amplification on a microarray of gel-immobilized oligonucleotides: Detection of bacterial toxin- and drug-resistant genes and their mutations." BIOTECHNIQUES, Bd. 29, Nr. 4, Oktober 2000 (2000-10), Seiten 844-857, XP002167651 ISSN: 0736-6205

## Beschreibung

Die vorliegende Erfindung betrifft ein allgemeines Verfahren zur spezifischen Bestimmung von DNA-Sequenzen mittels paralleler Amplifikation durch verschachtelte Polymerasekettenreaktion (im folgenden auch PCR = engl. "Polymerase Chain Reaction") in einem kombinierten Flüssigphasen/Festphasen-DNA-Microarraysystem (ein solches Festphasen-Microarraysystem wird unabhängig vom konkret daran immobilisierten Biomolekül (hier DNA) allgemein auch als "Biochip" bezeichnet). Ausgehend von diesem allgemeinen Verfahren, das als solches bereits den Nachweis bestimmter Analyten in einer zu analysierenden Probe gewährleistet, betrifft die vorliegende Erfindung gleichermaßen davon abgeleitete Verfahren zur Bestimmung von Punktmutationen und allelischen Varianten sowie Verfahren zur Bestimmung der Sequenz von DNA-Teilbereichen, und zwar auch von unbekannten DNA-Teilbereichen.

Im Rahmen der vorliegenden Beschreibung wird der Begriff "parallel" kontextabhängig mit zwei unterschiedlichen Bedeutungen verwendet. Zum einen ist z.B. die unten erwähnte parallele Bestimmung der einzelnen Sequenzbausteine durch Verwendung unterschiedlicher Primer möglich, d.h. der Multiplexbetrieb, zum anderen bedeutet "parallele" Amplifikation unter konkreter Bezugnahme auf ein erfindungsgemäßes Verfahren die gleichzeitige Amplifikation von Nucleinsäuren sowohl in der flüssigen als auch an der festen Phase mittels erfindungsgemäß bezeichneter P₃-Primer.

Im folgenden werden die Begriffe Sonde, PCR-Primer bzw. Primer je nach der Funktion des betreffenden Oligonucleotids verwendet. Ein Oligonucleotid, das als Primer zur spezifischen Amplifikation von Zielsequenzen verwendet werden kann, kann auch als Sonde zum Nachweis der Zielsequenz verwendet werden.

Ein Festphasen-Microarraysystem und ein Verfahren zur Analyse von markierten DNA-Sequenzen durch Hybridisierung an auf einem festen Träger immobilisierte Sonden und durch Bestimmung der Lokalisierung der Markierung auf der Oberfläche ist beispielsweise aus EP 0 373 203 bekannt.

Für eine solche DNA-Microarray-Analyse sind eine Reihe von Einzelschritten nötig. So muß die Proben-Nucleinsäure direkt oder in Form einer Kopie markiert werden, oder aber es wird mit einer Fänger-Nucleinsäure auf dem Mikroarray und einer zweiten Detektionssonde in Lösung gearbeitet. Dabei ist für viele Anwendungen, wie z.B. den Nachweis von biologischen Kontaminationen, die Amplifikation der Probensequenz durch z.B. eine PCR nötig. Die (markierte) Probe wird dann mit der auf dem Mikroarray gebundenen Sonde hybridisiert, um ein spezifisches, ortsgebundenes Signal zu erzeugen, welches z.B. über eine Detektionssonde aufgenommen und weitergeleitet werden kann. Durch diese Vorgehensweise fallen viele Einzelreaktionsschritte an, die diese Analysen arbeits-, kostenintensiv, langsam und darüber hinaus fehleranfällig machen.

Die Durchführung einer PCR in Anwesenheit eines festen Trägers bzw. Chip ("PCR on Chip") ist an sich bereits bekannt (Shoffner M. A., Cheng J., Hvichia G. E., Kricka U., Wilding P.: Chip PCR. I. Surface passivation of microfabricated silicon-glass chips for PCR. Nucleic Acids Res. 1996 Jan 15; 24(2):375-9.; Cheng J., Shoffner M. A., Hvichia G. E., Kricka U., Wilding P.: Chip PCR. II. Investigation of different PCR amplification systems in microfabricated silicon-glass chips. Nucleic Acids Res. 1996 Jan 15; 24(2):380-5.

Auch das Prinzip der sog. verschachtelten PCR (engl. "nested PCR") ist bekannt (vgl. z.B. C. R. Newton, A. Graham: "PCR", Spektrum Akademischer Verlag Heidelberg Berlin Oxford 1994, S. 59 und die dort angegebenen weiteren Literaturstellen). Bei der verschachtelten PCR werden zwei PCR-Primer-Paare verwendet, von denen die Mitglieder eines Paares (die sog. äußeren PCR-Primer) stromaufwärts bzw. stromabwärts der zu amplifizierenden Ziel-DNA-Sequenz hybridisieren. Die Mitglieder des zweiten Primer-Paares (die sog. inneren PCR-Primer) hybridisieren innerhalb des Abschnitts, der vom Design des ersten Primer-Paares vorgegeben wird. Der Vorteil dieser Technik besteht in der erheblichen Erhöhung der Empfindlichkeit und der Spezifität, da bevorzugt die Ziel--DNA-Sequenz amplifiziert wird. Diese Technik ist aber außerordentlich empfindlich gegenüber Verunreinigungen, wie sie beispielsweise auch durch das Zusammenpipettieren der für die PCR erforderlichen Reagenzien eingeschleppt werden können.

Außerdem muß bei einer verschachtelten PCR entweder das erste Amplikon verdünnt oder mindestens einer der äußeren Primer entfernt werden. Wenn alle drei Primer gleichzeitig in dem Ansatz vorliegen, ergibt sich keine Erhöhung der Empfindlichkeit und der Spezifität mehr. Ein Hinzupipettieren ist nach der ersten Amplifikation im Prinzip zwar möglich, jedoch mit dem Risiko verbunden, daß die der "nested PCR" normalerweise innewohnenden Vorteile (s.o.) nicht realisiert werden können.

Im allgemeinen wird die Verdünnungsmethode angewendet, bei der die Konzentration der ursprünglichen Matrize und die Konzentration mindestens eines der äußeren Primer (nachfolgend mit P₁ bzw. P₂ bezeichnet) stark vermindert wird.

Es ist ferner versucht worden, die PCR-Reagenzien vorzumischen und entsprechend zu stabilisieren, insbesondere um die Durchführung der PCR-Reaktion zu vereinfachen. Im Stand der Technik ist jedoch nicht beschrieben, dass die Reaktionsteilnehmer der vorgesehenen Umsetzung direkt auf einer Microarrayanordnung in dehydratisierter Form bereitgestellt werden, so daß die Reaktion nur noch durch Zugabe der vorzugsweise wäßrigen Probenlösung gestartet werden kann.

Im Stand der Technik sind unterschiedliche Techniken zur Lyophilisierung bzw. Trocknung von Reagenzien für die Durchführung von Nucleinsäure-Analysen beschrieben. So wird im US-Patent 5 565 318 die Herstellung von Semisphären mit allen für eine Reaktion nötigen Komponenten, darunter auch Nucleinsäuren, beschrieben. Ähnliche Techniken werden in den Anmeldungen FR 2 674 253, EP 0 298 669, EP 0 726 310, GB 2 333 358, WO 94/17106, WO 98/00530, US 5 250 429 und EP 0 383 569 angewendet.

In der Patentanmeldung WO 96/32497 werden konkret alle wichtigen Nucleinsäure-Techniken in Bezug auf die Verwendung solcher Reagenziensphären beschrieben. In der Patentanmeldung WO 94/16107 ist zusätzlich noch ein Apparat bzw. eine Vorrichtung erwähnt, bei dem die getrockneten Reagenzien zu einer Sequenzierungsreaktion auf einer Platte (vermutlich einer Microtiterplatte) verwendet werden. In der Patentanmeldung WO 96/17083 wird ein fester Träger in Form eines Kammes beschrieben, auf dem sich diese Reagenzien befinden und der auf sog. 8-ter Strips bzw. Microtiterplatten gegeben werden kann, so daß nach der Ablösung der Reagenzien die Reaktion in den Vertiefungen der Microtiterplatten ablaufen kann.

In der Patentanmeldung WO 93/14223 wird die Aufbringung der einzelnen PCR-Reagenzien in Form von räumlich voneinander getrennten Flecken ("Spots") bzw. auch eine flächige Aufbringung an eine feste Phase in Form eines Objektträgers offenbart. Ferner wird die reversible Bindung getrockneter Markierungssubstanzen und die Markierung einer Sonde nach der Rehydrierung beschrieben. Die Sonde bzw. Sonden-Nucleinsäure selbst liegt hierbei aber ausdrücklich reversibel an den festen Träger gebunden vor.

Der Artikel von Head et al., "Nested Genetic bit analysis for mutation detection in the p53 tumor surpressor gene, nucleic acid research, Oxford University Press, Surrey, Great Britain, vol. 25, Nr. 24, 1997, S. 5065, 5071, offenbart ein Verfahren zur Bestimmung von DNA-Sequenzen mittels Amplifikation unter Verwendung von "in Arrays" vorliegenden immobilisierten Primern sowie die Verwendung einer verschachtelten PCR-Reaktion.

Die WO97/32040 offenbart ein Verfahren zur Sequenzdetektion bei Nukleinsäuren, bei dem eine verschachtelte Flüssig-/Festphasen-PCR mit zwei äußeren Primern sowie einem irreversible an eine Microtiterplatte gebundenen Primer zum Einsatz kommt. Das Extensionsprodukt wird durch an die immoblisierten Primer gebundene markierte Primer bestimmt. Das Dokument offenbart einen sequenziellen Reaktionsablauf der PCR mit nachgeschalteter Hybridisierung am Array, so dass keine gleichzeitige verschachtelte PCR-Reaktion erfolgt.

Die WO98/28438 offenbart ein Verfahren zur Bestimmung von wenigstens einem Nukleotid, das auf der Immobilisierung von "Arrays" aus Oligonukleotid-Primern, deren Hybridisierung mit einer Zielsequenz, anschließender Extension sowie Waschen und Detektion beruht, wobei die Verfahrensschritte Hybridiseren, Extension und Denaturierung mehrfach ausgeführt werden können. Die Druckschrift offenbart zwar einen Ansatz, bei dem alle Reagenzien gleichzeitig hinzugegeben werden, allerdings findet auch hier keine gleichzeitige verschachtelte Reaktion an der Festphase und in der flüssigen Phase statt.

Keine dieser im Stand der Technik beschriebenen Techniken ermöglicht die parallele Amplifikation in einem gekoppelten Flüssig-/Festphasen PCR-System, bei dem die gewünschten Ergebnisse durch Auswertung eines direkt an der festen Phase des Systems nachweisbar gebildeten und damit kovalent gebundenen Extensionsprodukts hoch-spezifisch, mit hohem Durchsatz und reproduzierbar - gewünschtenfalls auch in Echtzeit - generiert und den unterschiedlichen Anwendungsgebieten zugeführt werden können.

Aufgabe der Erfindung ist, die dem Stand der Technik anhaftenden Mängel zu beseitigen und ein einfaches und fehlerfrei zu handhabendes und preisgünstiges verfahren zur Amplifikation und Analyse von DKA-Sequenzen bereitzustellen, ohne das Kompromisse hinsichtlich der Empfindlichkeit und Spezifität zu machen sind.

Erfindungsgemäß wird diese Aufgabe gelöst durch ein allgemein anwendbares Verfahren zur spezifischen Bestimmung von DNA-Sequenzen mittels paralleler Amplifikation in einem kombinierten Flüssigphasen/Festphasen-DNA-Microarraysystem, indem man eine verschachtelte Polymerasekettenreaktion zur gleichzeitigen Amplifikation von Nukleinsaüren im des flüssigen und des festen Phase unter Verwendung von x PCR-Primersätzen aus jeweils mindestens 3 PCR-Primern P₁1, P₂1, P₃1, P₁2, P₂2, P₃2, P₁3, P₂3, P₃3, ... P₁x, P₂X, P₃X durchführt, wobei x eine natürliche Zahl bedeutet und der Anzahl der zu bestimmenden DNA-Sequenzen entspricht, und wobei für jeden der.x PCR-Primersätze
(a) zwei äußere PCR-Primer P₁, P₂ in des flüssigen Phase vorliegen und so ausgewählt sind, daß sie an stromaufwärts und stromabwärts der zu amplifizierenden Ziel-DNA-Sequenz liegende DNA-Teilsequenzen hybridisieren,
(b) ein innerer PCR-Festphasen-Primer P₃ so ausgewählt wird, daß er an eine innerhalb der zu bestimmenden Ziel-DNA-Sequenz liegende DNA-Teilsequenz hybridisiert und in der Lage ist, ein P₃₋Extensionsprodukt zu bilden, und
(c) die x äußeren PCR-Primer P₁1, P₂1, P₁2, P₂2, P₁3, P₂3, ... P₁x, P₂x in der flüssigen Phase gegenüber den x inneren PCR-Primern P₃1, P₃2, P₃3, ... P₃x im Überschuß vorliegen, und
(d) die x inneren PCR-Primer P₃1, P₃2, P₃3, ... P₃x an x räumlich beabstandeten Positionen unter Bildung eines DNA-Microarrays irreversibel an eine Festphase gebunden vorliegen, und
(e) man die Bestimmung durch Ermittlung des Vorhandenseins eines P₃-Extensionsproduktes an der definierten Position des DNA-Microarrays durchführt.

Bevorzügte Ausführungsformen des Verfahrens sind in den angefügten Ansprüchen dargestellt.

Mit dem erfindungsgemäßen Verfahren lassen sich u.a. die nachfolgend dargelegten Vorteile erzielen. Die verschachtelten PCR-Reaktionen laufen direkt und parallel auf dem Festphasen-DNA-Mikroarray ab. Das bei der Durchführung einer konventionellen verschachtelten PCR in flüssiger Phase üblicherweise erforderliche Verdünnen und Neueinstellen des Reaktionsansatzes nach der ersten PCR unter Verwendung der äußeren Primer P₁ und P₂ entfallen. Für den Anwender des vorliegend beschriebenen Verfahrens fällt zur Durchführung der gesamten Analyse nur noch ein Arbeitsschritt an, d.h. er muß nur noch die Proben-Nucleinsäuren und die PCR-Reagenzien (DNA-Polymerase und Desoxynucleosidtriphosphate in PCR-Puffer) zupipettieren, sofern diese Reagenzien vollständig oder zum Teil nicht bereits nach einer bevorzugten Ausführungsform in dehydratisierter Form auf dem festen Träger bereitgestellt werden. Dadurch sinken sowohl der Arbeitsaufwand als auch die Kontaminationsgefahr signifikant bei deutlicher Reduktion der Fehlermöglichkeiten.

Das erfindungsgemäße Verfahren zur spezifischen Bestimmung von DNA-Sequenzen mittels paralleler Amplifikation durch verschachtelte PCR in einem kombinierten Flüssigphasen/Festphasen-DNA-Microarraysystem besitzt die gleiche Empfindlichkeit wie eine konventionelle, d.h. in flüssiger Phase durchgeführte, PCR und gleichzeitig eine höhere Spezifität als Hybridisierungsassays und Primer-Extensionassays. Der Grund dafür ist, daß die dem System Primer/Proben-DNA/Polymerase eigene Spezifität in bezug auf die Amplifikation zusätzlich noch durch die spezifische Wechselwirkung zwischen dem an den festen Träger immobilisierten inneren PCR-Primer (der somit auch die Funktion einer Sonde hat) und dem Amplikon deutlich erhöht wird. Insgesamt resultiert so eine Spezifität, die z.3. derjenigen eines 5'-Exonuklease-Assays (z.3. unter Verwendung von TaqMan™-Polymerase) überlegen ist.

Diese Vorteile lassen sich mit einem einfachen Festphasenprimer-Extensionassay, wie z.B. der Mikrotiterplatten-Festphasen-PCR (Nunc, vgl. z.B. NucleoLink™) oder der "Bridge™-PCR" (Mosaic Technologies, vgl. z.B. WO/36094), nicht erzielen.

Durch die ortsgebundenen inneren PCR-Festphasen-Primer, die auch als Sonden fungieren, kann nach einer bevorzugten Ausführungsform des Verfahrens der Erfindung auf dem Mikroarray mit einem Markierungsreagenz (beispielsweise unter Verwendung markierter Nucleotide) gearbeitet werden, um parallel viele unterschiedliche Sequenzen, Merkmale oder Eigenschaften zu untersuchen.

Eine markierungsfreie Detektion, beispielsweise mit optischen oder anderen physikalischen Methoden ist aber ebenfalls möglich, d.h. das erfindungsgemäße Verfahren kann auch ohne markierte Nukleotide durchgeführt und die Reaktionsprodukte gemessen werden.

Hier zeigt das erfindungsgemäße Verfahren gegenüber den konventionellen Hybridisierungsverfahren des Standes der Technik, bei denen unspezifische Massezuwächse an der Oberfläche des festen Trägers ein Problem darstellen, den Vorteil, daß der unspezifische Massezuwachs an der Oberfläche durch Anwendung hoher Temperaturen stark vermindert wird, wobei die kovalente Bindung der spezifischen Extensionssprodukte an den Festphasenprimer P₃ für eine nahezu ausschliessliche Bestimmung der gewünschten Spezifität sorgt.

Die Messung eines ortsspezifischen Massezuwachses kann z.B. durch physikalische Methoden erfolgen. Beispielsweise kann die ortsspezifische Änderung des Brechungsindexes, die ortsspezifische Änderung des elektrischen Widerstandes bzw, der elektrischen Leitfähigkeit, die ortsspezifische Änderung der optischen Dichte oder auch ortsspezifische dichroische Effekte, etc. gemessen werden.

Grundsätzlich ist das allgemeine erfindungsgemäße Verfahren für ein breites Spektrum an Anwendungsgebieten geeignet, wobei zwischen reinem diagnostischen Nachweis bestimmter Analyten in einer zu analysierenden Probe einerseits und komplexer abgeleiteten Modifikationen des Verfahrens zur Ermittlung von Sequenzdaten oder Informationen über funktionelle Zusammenhänge im Rahmen entsprechender Genomik- bzw. Proteomik-Fragestellungen unterschieden werden kann. Diese Unterscheidung dient jedoch nur der Veranschaulichung und soll die grundsätzlich offene Anwendbarkeit des erfindungsgemäßen Verfahrens in keiner Weise beschränken.

Nach einer besonderen Ausführungsform umfasst das allgemeine Verfahren der vorliegenden Erfindung daher ein davon abgeleitetes Verfahren insbesondere zur Bestimmung von Punktmutationen mittels paralleler Amplifikation, bei dem man
(a) unter Anwendung des oben definierten erfindungsgemäßen Verfahrens eine Polymerasekettenreaktion durchführt, durch welche sämtliche x zu bestimmenden Varianten amplifiziert werden, wobei
(b) mindestens P₃x Primerfamilien verwendet werden, wobei sich jedes einzelne Mitglied P₃x-N der Primerfamilie hinsichtlich des Nukleotids an seinem 3'-Ende von jedem anderen Mitglied unterscheidet, wobei N die unterschiedlichen Nukleotide A, C, G und T bezeichnet, und die Annealingtemperatur der Amplifikationsreaktion so ausgewählt wird, daß die gewünschten Extensionsprodukte nur an denjenigen Positionen des DNA-Microarrays gebildet werden, welche durch die Anwesenheit des zu der zu bestimmenden Punktmutation im wesentlichen exakt komplementären Primers gekennzeichnet sind,
(c) zur Amplifikation eine DNA-Polymerase ohne 3'→5' -Exonuclease-Aktivität verwendet wird.

Die Angabe "im wesentlichen exakt komplementärer Primer" bedeutet hier, daß hybridisierte und von der DNA-Polymerase als Matrize (engl. "Template") akzeptierte Primer verlängert werden, d.h. in der Regel sind im Bereich des 3'-Endes sämtliche Nukleotide komplementär zur Matrize.

Ferner umfasst das allgemeine Verfahren der vorliegenden Erfindung ein davon abgeleitetes Verfahren zur Bestimmung der Sequenz von DNA-Teilbereichen mittels paralleler Amplifikation, bei dem
(a) unter Anwendung des oben definierten erfindungsgemäßen Verfahrens eine Polymerasekettenreaktion durchgeführt wird, durch welche sämtliche x zu bestimmenden Sequenzen amplifiziert werden, wobei
(b) mindestens P₃x Primerfamilien verwendet werden, wobei sich jedes einzelne Mitglied P₃x-N,... P₃x-Nn der Primerfamilie hinsichtlich seiner 1 bis n Nukleotide am 3'-Ende von jedem anderen Mitglied unterscheidet, wobei N die unterschiedlichen Nukleotide A, C, G und T bezeichnet, und n die Länge der zu bestimmenden Sequenz(en) angibt, und die Annealingtemperatur der Amplifikationsreaktion so ausgewählt wird, daß die gewünschten Extensionsprodukte nur an denjenigen Positionen des DNA-Microarrays gebildet werden, welche durch die Anwesenheit des zu der zu bestimmenden Sequenz im wesentlichen exakt komplementären Primers gekennzeichnet sind,
(c) zur Amplifikation eine DNA-Polymerase ohne 3'→5'-Exonuclease-Aktivität verwendet wird.

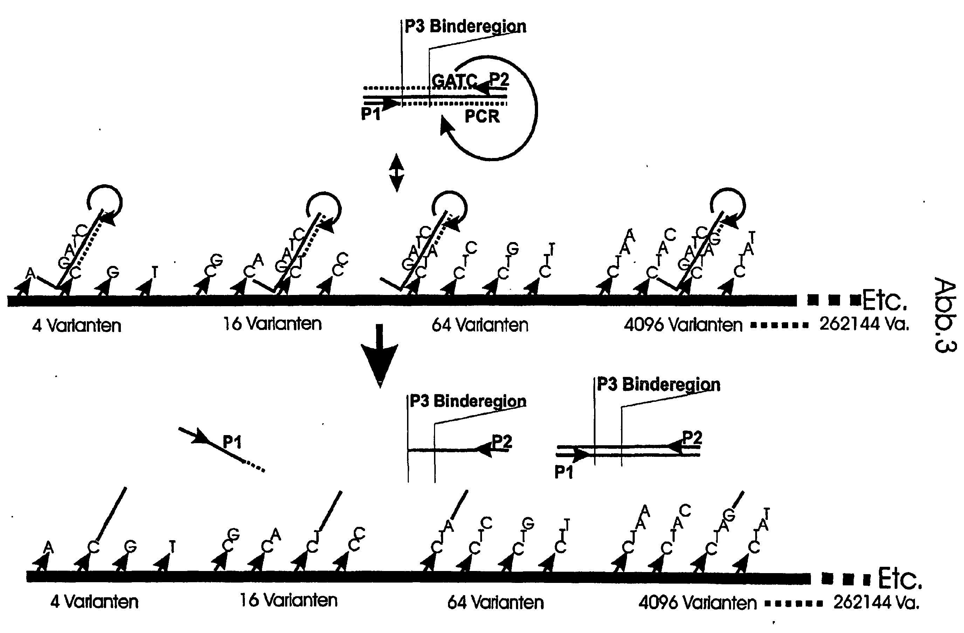

Obwohl sich die Anwendungsbereiche der ersten beiden abgeleiteten Modifikationen des allgemeinen erfinderischen Verfahrens durchaus unterscheiden können, erfolgt die Ermittlung im wesentlichen gleich. Zwar soll im abgeleiteten verfahren zur Bestimmung der Sequenz von DNA-Teilbereichen mehr als nur das endständige 3'-Nukleotid bestimmt werden, aber durch das bekannte Design des Mikroarrays und die Gruppierung der einzelnen Reaktionsbereiche in Felder, die mit der ersten, zweiten, dritten ... bzw. n-ten Position der zu bestimmenden Sequenz korrespondieren, erfolgt die Bestimmung des DNA-Teilbereichs durch sukzessives Ablesen der für jedes Feld erhaltenen Daten. Mit anderen Worten wird zuerst das Feld ausgewertet, in welchem ausgehend vom Design des Arrays die erste zu bestimmende Base ermittelt wird, bevor in Kenntnis des Ergebnisses aus diesem ersten Feld die Bestimmung der Base an der zweiten Position der zu ermittelnden Sequenz vorgenommen wird, u.s.w. Die jeweilige Untersuchung eines Feldes erfolgt vorzugsweise durch Erfassung und Zuordnung der größten gemessenen Signalstärke. Selbstverständlich können die Feldmessungen auch parallelisiert ablaufen. Ergänzend wird in diesem Zusammenhang auf Abb. 3 verwiesen.

Nach einer weiteren bevorzugten Ausführungsform der vorliegenden Erfindung umfasst das allgemeine Verfahren ferner ein Verfahren zur Bestimmung der Sequenz von unbekannten DNA-Teilbereichen mittels paralleler Amplifikation, bei dem unter Anwendung des oben definierten allgemeinen erfindungsgemäßen Verfahrens eine Polymerasekettenreaktion durchgeführt wird, durch welche die Abfolge sämtlicher n Nukleotide der zu bestimmenden Sequenzen ermittelt wird, wobei
(a) ein sämtliche Permutationen umfassender Satz von inneren Primern P₃ mit einer bestimmten Länge n verwendet wird, wobei mehrere Primer mit einer jeweils unterschiedlichen Region der zu bestimmenden Sequenz exakt hybridisieren und Extensionsprodukte definierter Länge bilden können, und sich jeder einzelne dieser Primer an einer anderen Position des DNA-Microarrays befindet,
(b) zur Amplifikation eine DNA-Polymerase ohne 3'→5'-Exonuclease-Aktivität verwendet wird, und
(c) die Bestimmung der Sequenz durch Ermittlung der in Schritt (a) gebildeten Extensionsprodukte, Zuordnung dieser ermittelten Werte zu den Positionen des DNA-Microarrays, und Zusammenstellung der zu bestimmenden Sequenz im Rahmen einer ggf. Computer-unterstützten Kombinatorik erfolgt, wobei die tatsächliche Aneinanderreihung der Einzeldaten zur Gesamtsequenz unter Nutzung der jeweils überlappenden Sequenzbereiche vorgenommen wird.

Alternativ kann eine Konsensus-PCR auch durch das Anligieren von PCR-Adaptoren erfolgen. Damit wird eine Signatur-Sequenzierung ausgehend von einem "Pool" an cDNA Sequenzen erreicht (auch quantitativ ausführbar im Falle einer Online-Beobachtung der Amplifikation). Ein solches Sequenzierungsverfahren (MPSS = engl. "Massively Parallel Signature Sequencing") wurde kürzlich beschrieben (Brenner et al., Nature Biotechnology Vol. 18 June 2000, Seiten 630-34) und kann unter Berücksichtigung der vorliegend offenbarten technischen Lehre auf sehr viel einfachere Weise durchgeführt werden. Das erfindungsgemäße Verfahren erfordert hierzu lediglich die cDNA-Generierung, einen Restriktionsverdau und die Adaptorligation vor dem Assay. MPSS erfordert im Gegensatz dazu die cDNA-Herstellung, einen Restriktionsverdau, die anschließende Hybridisierung an "Beads", die "FACS"-Sortierung (FACS = engl. Fluorescence-activated Cell Sorter) der "Beads" und eine Wiederholung der Ligation sowie Restriktionsverdauschritte.

Dem Fachmann sind die grundsätzlichen Schritte der Sequenzermittlungen aus der allgemeinen Literatur zur "Sequencing by Hybridization" -Technologie bekannt (vgl. z.B. EP 0 373 203).

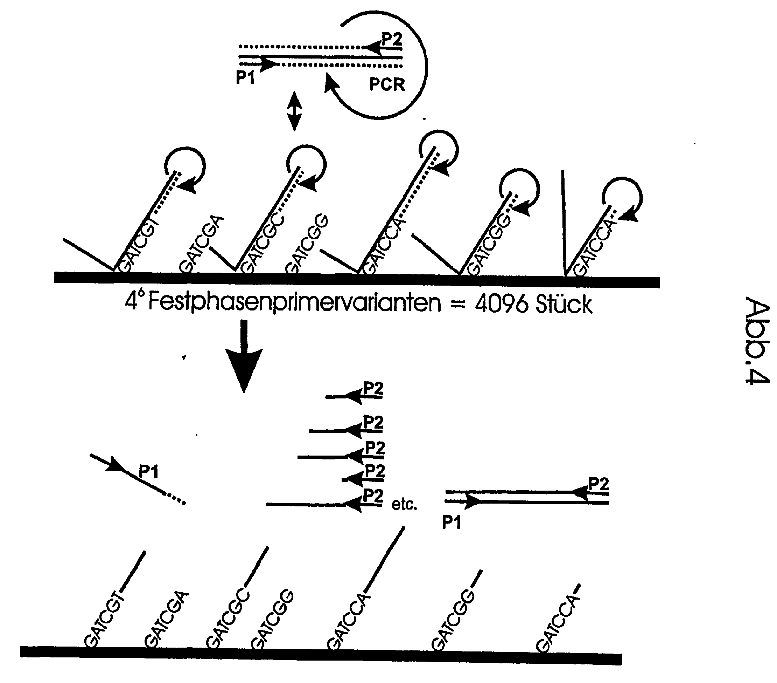

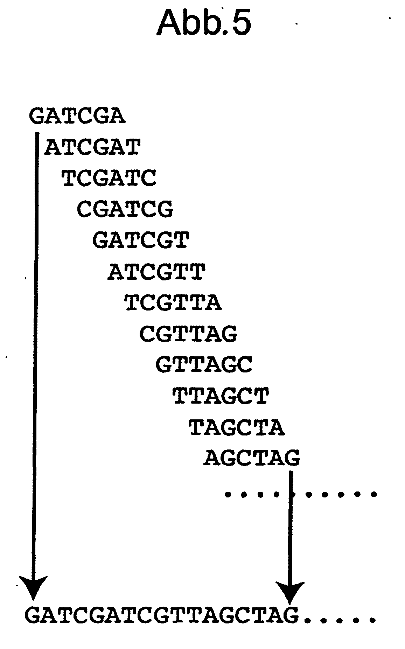

Im übrigen wird zur Veranschaulichung des geschilderten Verfahrens auf die Abbildungen 4 und 5 verwiesen.

Nach einer alternativen Ausführungsform dieses Verfahrens erfolgt die Bestimmung der Sequenz durch Ermittlung der jeweiligen Längen der in Schritt (b) gebildeten Extensionsprodukte und Zuordnung der ermittelten Werte zu den definierten Positionen des DNA-Microarrays.

Nach einer weiteren bevorzugten Ausführungsform kann das zuvor beschriebene Verfahren auch zur Signatur-Sequenzierung und Quantifizierung ausgehend von einer unbekannten Sequenz mittels Primern erfolgen, welche mindestens 9 Nukleotide aufweisen.

Unbekannte Sequenzen werden hierzu mit einem Restriktionsenzym geschnitten, Adaptorprimer anligiert und die Ligationsprodukte von der restlichen DNA separiert und z.B. mittels Nonamerprimern auf und an dem Chip amplifiziert. Zum Beispiel werden die cDNAs eines kompletten bakteriellen RNA-"Pools" bestimmt. Das Amplikon umfaßt vorzugsweise 18-25 bp. Die Festphasenprimer sind alle 9 Nucleotide lang und unterscheiden sich in ihrer Sequenz. Es sind 262144 unterschiedliche Primervarianten auf der Festphase. Wiederum werden Standard-PCR-Bedingungen angewandt, aber die Annealingtemperatur wird nach 10-20 Zyklen auf 30-50°C erniedrigt, um die Festphasen-Amplifikation mit den Nonameren zu begünstigen. Nur exakt komplementäre Primer werden verlängert. Das entstandene Muster nach Anfärbung erlaubt durch Abgleich mit üblichen Datenbanken die Sequenzbestimmung.

Somit wird die Sequenzermittlung von Produkten des "SAGE"-Verfahrens ermöglicht (Velculeecu V. E. et al., Science 1995, Vol. 270, Seiten 484-87; Neilson, L. et al., Genomics 2000, Vol 63, Seiten 13-24) Durch Online-Detektion kann die Ausgangsmenge der cDNAs bestimmt werden.

Außerdem betrifft die vorliegende Erfindung einen Festphasen-DNA-Microarray zur Durchführung eines erfindungsgemäßen Verfahrens, auf dem an n räumlich beabstandeten Positionen x innere PCR-Primer P₃1, P₃2, P₃3, ... P₃x irreversibel an die Festphase gebunden sind.

Durch das erfindungsgemäße Verfahren zur Bestimmung der Sequenz von DNA-Teilbereichen mittels paralleler Amplifikation lassen sich die Nachteile der Sequenzierungsverfahren des Standes der Technik beheben, nämlich unter anderem die durch die grundsätzliche Linearität einer solchen Methodik vermittelte geringe Empfindlichkeit.

Beispielsweise findet beim sog. "Cycle Sequencing" nur eine Amplifikation in der flüssigen Phase statt, so daß die Trennung der Sequenzierungsprodukte nötig ist, was Fehlermöglichkeiten beinhaltet und arbeitsaufwendig ist.

Beim sog. "Mini Sequencing" (nach der Sanger' schen Dideoxy-Sequenziertechnik) muß dagegen die Matrize bzw. das "Template" in großer Menge verwendet werden, da nur Dideoxynucleotide (ddNTPs) verwendet werden, so daß lediglich eine lineare Amplifikation stattfindet.

Beide beispielhaft beschriebenen Sequenzierungstechniken weisen gegenüber dem erfindungsgemäßen Verfahren den Nachteil auf, dass eine gleichzeitige, d.h. parallele Amplifikation der zu bestimmenden Matrize nicht möglich ist.

Das erfindungsgemäße Verfahren ist im übrigen dadurch von den bekannten Verfahren wie insbesondere der "Sequencing-by-Hybridisation"-Technik (wie z.B. in EP 0 373 203 beschrieben) abgegrenzt, daß nicht ein gesamtes Amplikon, sondern gezielt nur relevante Bereiche davon sequenziert werden können. Darüber hinaus ermöglicht das Verfahren eine gleichzeitige Bestimmung der Sequenz mehrerer interner Regionen des Amplikons.

Zur SNP-Ermittlung (engl. "Single Nucleotide Polymorphism") durch Hybridisierung oder unter Anwendung der ARMS-Technik (engl. "Amplification Refractory Mutation System", vgl. z.B. P. Scheinert: Nachweis von Mutationen; BioTec Labortechnik, 5/6/1998) muß die betreffende Sequenz bekannt sein. Außerdem wird ein sog. "Fehl-Priming" nicht erkannt. Ein Multiplexbetrieb (parallele Durchführung unter Verwendung mehrerer verschiedener Primer) ist aufgrund der Generierung von Extensionsprodukten gleicher Länge nicht möglich, so daß getrennte Ansätze zwingend sind.

Das erfindungsgemäße Verfahren zur Bestimmung von Punktmutationen mittels paralleler Amplifikation hat demgegenüber insbesondere die nachfolgend dargestellten Vorteile.

Die Sequenzen der zu ermittelnden 3'-Basen müssen nicht (können aber) bekannt sein. Außerdem müssen keine terminierenden Agenzien verwendet werden (wie bei der Sanger'schen Dideoxy-Sequenziertechnik), und es ist die parallele Bestimmung der einzelnen, verschiedene SNP differentiell charakterisierenden, Sequenzbausteine möglich, d.h. der Multiplexbetrieb.

Die Empfindlichkeit ist sehr hoch (1 Kopie wird erkannt), da mit minimalsten Matrizenmengen (1 Kopie reicht) mit Hilfe exponentieller Amplifikation sequenziert wird.

Weitere vorteilhafte und/oder bevorzugte Ausführungsformen der Erfindung sind Gegenstand der Unteransprüche.

Im folgenden wird die Erfindung ohne Beschränkung anhand von Ausführungsbeispielen und unter Bezugnahme auf die in den Figuren dargestellten Abbildungen detaillierter beschrieben.

### In den Figuren zeigt:

Abb. 1: den spezifischen Nachweis einer bekannten Sequenz.

Abb. 2: eine Allotypbestimmung durch spezifische Bestimmung der Identität eines 3'-ständigen Nucleotids mittels Primerfamilien.

Abb. 3: eine sog. "Sequence Tag Aquisition", d.h. eine spezifische Signatur-Sequenzierung ausgehend von einer bekannten Primersequenz mittels Primerfamilien.

Abb. 4: eine Signatur-Sequenzierung ausgehend von einer unbekannten Sequenz mittels Primer-Hexameren.

Abb. 5: das Zusammenführen der durch die Signatur-Sequenzierung gemäß Abb. 4 erhaltenen Einzelergebnisse zur Generierung einer kontinuierlichen Sequenzabfolge.

Bei einer vorteilhaften Ausgestaltung des erfindungsgemäßen Verfahrens zur spezifischen Bestimmung von DNA-Sequenzen mittels paralleler Amplifikation liegen die x äußeren PCR-Primer P₁1, P₂1, P₁2, P₂2, P₁3, P₂3, ... P₁x, P₂x gegenüber den x inneren PCR-Primern P₃1, P₃2, P₃3, ... P₃x in einem 10² - bis 10¹² -fachen, z.B. in einem 10⁴-fachen, Überschuß vor. Dadurch läuft die PCR am Anfang verstärkt in der flüssigen Phase ab. Mit zunehmender Amplikon-Konzentration findet die PCR aber zunehmend auf der Festphasenoberfläche statt und entspricht dort einer verschachtelten PCR.

Bei einer weiteren vorteilhaften Ausgestaltung des erfindungsgemäßen Verfahrens zur spezifischen Bestimmung von DNA-Sequenzen mittels paralleler Amplifikation sind die Schmelztemperaturen (Tm) der zwei äußeren PCR-Primer P₁ und P₂ gegenüber derjenigen des inneren Primers P₃ unterschiedlich. Beispielsweise haben die äußeren PCR-Primer eine höhere Schmelztemperatur als der innere PCR-Primer. Der Reaktionsverlauf kann dann so eingestellt werden, daß zunächst bei erhöhter Temperatur nur die äußeren PCR-Primer binden bzw. "annealen" und verlängert werden. Nach der entsprechenden PCR-Zyklenzahl wird dann die Temperatur gesenkt, so daß auch der innere PCR-Primer an sein "Template" binden und die für die Bestimmung gewünschten Extensionsprodukte bilden kann.

Auch die verschiedenen Festphasenprimer P₃ untereinander können unterschiedliche Schmelztemperaturen aufweisen. Daher ist es möglich, die Reaktion so zu steuern, daß nur an bestimmten definierten Positionen des DNA-Microarrays eine Amplifikation stattfindet. Durch die Erstellung von Amplifikationsprofilen können gewünschtenfalls die unterschiedlichen Eigenschaften der Festphasenprimer wie z.B. Basenzusammensetzung, G-C Gehalt und Länge berücksichtigt werden.

Bei dem erfindungsgemäßen Verfahren zur Bestimmung von DNA-Sequenzen mittels paralleler Ampifikation unterliegt die Festphase bzw, der feste Träger keinen besonderen Beschränkungen und kann beispielsweise unter Metall(z.B. Aluminium oder Gold)oberflächen, mit SiO₂ bedampften Metalloberflächen, Metall/Halbmetalloxid(z.B. Al₂O₃ oder SiO₂) oberflächen, Glasoberflächen, Polymeroberflächen, x.B. in Folienform, Nylonmembranen oder Nitrocellulosemembranen ausgewählt werden. Dem Fachmann ist aber klar, daß sich grundsätzlich auch "halbfeste" oder gelartige Träger eignen. Wichtig ist nur, daß eine örtliche Fixierung bzw. räumliche Lokalisierung der Nachweisreaktion möglich ist.

Ein erfindungsgemäß bevorzugt geeigneter gelartiger Träger kann z.B. hergestellt werden, indem auf einem herkömmlichen festen Träger an sich bekannte Polymerisationsinitiatoren immobilisiert werden, an die dann entsprechende Monomere unter Bildung von sog. "Polymer Brushes" polymerisiert werden. Diese Monomeren können zum einen funktionelle Gruppen aufweisen, über die eine Vernetzung der "Polymer Brushes" miteinander möglich ist, und zum anderen funktionelle Gruppen haben, die zur Immobilisierung von Sonden-Molekülen, z.B. Oligonucleotiden oder Antikörpern, dienen (Linker-Gruppen). In wäßriger Phase quillt die Schicht aus "Polymer Brushes" auf der Oberfläche des festen Trägers gelartig auf. Mit "Polymer Brushes" lassen sich erheblich höhere Propfdichten auf der Trägeroberfläche erreichen als unter Verwendung der konventionellen, sich selbst organisierenden Monoschichten (engl. "Selfassembled Monolayers", "SAMs") aus bifunktionellen Molekülen (engl. "Linker"). Auch die Kopplungsdichte in bezug auf die Proben- oder Sondenmoleküle ist erheblich höher. Einzelheiten zur "Polymer-Brush"-Technik sind in der WO 00/43539 beschrieben, auf die hier ausdrücklich Bezug genommen wird.

Bevorzugt wird Glas verwendet, da es keine poröse Oberfläche hat, in deren Poren die Ziel-DNA diffundieren könnte und dadurch langsamer ihre entsprechende Sonde findet. Außerdem ist Glas mechanisch stabil, temperaturbeständig, unempfindlich gegenüber stringenten Waschbedingungen und hat eine geringe Eigenfluoreszenz. Es sind alle Glasarten oder -sorten geeignet, z.B. Quarzglas.

Die Polymeroberfläche kann z.B. aus Polypropylen, Polymethylmethacrylat (PMMA) (Acryl- bzw. Plexiglas) oder Cycloolefin-Copolymeren (COCs) bestehen. Ein geeignetes COC ist beispielsweise von Ticona unter dem Handelsnamen "Topaz" erhältlich.

Die genauen Sequenzen der äußeren und inneren Primer bzw. Sonden wählt der Fachmann nach Maßgabe des konkreten analytischen Problems. Beispielsweise kann es sich um Sequenzen handeln, die an für die nachzuweisenden oder zu differenzierenden Mikroorganismen spezifische DNA-Sequenzen hybridisieren. Organismenspezifische Sequenzen können beispielsweise durch Sequenzdatenbankvergleiche und ggf. "Alignment" ermittelt werden. Grundsätzlich besteht keine Beschränkung auf DNA oder allgemein Nucleinsäuren als Sonden. Es können wegen der bekannten Vorteile auch DNA-PNA(Peptidnucleinsäure)-Hybride bzw. -Chimäre verwendet werden. Ferner können auch modifizierte Nucleotide (z.B. dI, dI-Biotin, dU, dU-Biotin) eingesetzt werden.

Die direkte oder indirekte Immobilisierung der Biomoleküle mit oder ohne Spacer an der Oberfläche des festen Trägers erfolgt erfindungsgemäß durch kovalente Bindung und betrifft gleichermaßen sowohl die in situ-Synthese von z.B. Oligonucleotiden als auch das Aufpropfen zuvor synthetisierter Nucleotidfolgen unabhängig von der jeweiligen Länge und tatsächlichen Beschaffenheit der als Sonden und/oder Primer dienenden Nucleinsäuren.

Beispielsweise kann ein Glasträger in eine Lösung von bifunktionellen Molekülen (sog. "Linker"), die z.B. eine Halogensilan- (z.B. Chlorsilan-) oder Alkoxysilangruppe zur Kopplung an die Glasoberfläche aufweisen, getaucht werden.

Das verwendbare bifunktionelle Silan unterliegt keinen besonderen Beschränkungen. In Frage kommen alle Silane, die am Siliciumatom ein(e), zwei oder drei hydrolysierbare(s) Atom(e) oder Gruppe(n) aufweisen, beispielsweise Halogenatome, C₁-C₄₋Alkoxy-, C₁-C₄-Acyloxy- oder Aminogruppen.

Konkret kann zum Beispiel Isocyanopropyltrimethoxysilan oder 3-Glycidoxypropyltrimethoxysilan auf einen Glasträger aufgebracht werden.

Die zweite funktionelle Gruppe des bifunktionellen Silans unterliegt ebenfalls keinen besonderen Beschränkungen und wird nach Maßgabe des zu immobilisierenden Proben- oder Sondenmoleküls (z.B. Nucleinsäuren) gewählt. Beispiele sind zu nucleophilen Substitutionsreaktionen, nucleophilen Additionsreaktionen, Diels-Alder-Reaktionen oder radikalischen Substitutionsreaktionen fähige reaktive Gruppen. Konkrete Beispiele sind reaktive Doppelbindungen, Diengruppen, dienophile Gruppen, Epoxy-, Aldehyd- Hydroxy-, Carbonsäure-, Aktivester-, Amino, Disulfid- Thiol-. Aziridin- Azlacton-, Isocyanat-, Amino, Disulfid-, Thiol-, Aziridin-, Azlacton-, Isocyanat-, Isothiocyanat-, Azidgruppen und reaktive Austrittsgruppen.

Diese und weitere bifunktionelle Linker für die Kopplung einer Vielzahl von Proben- oder Primer/Sonden-Molekülen, insbesondere auch biologischen Ursprungs, an eine Vielzahl von Trägeroberflächen sind dem Fachmann gut bekannt, vgl. beispielsweise "Bioconjugate Techniques" von G. T. Hermanson, Academic Press 1996.

Für Nylonmembranen können z.B. Oligonucleotide mit Oligo-(dT)-Schwanz am 5'-Ende verwendet werden. Durch UV-Bestrahlung werden die Thymin-Basen des Oligo-(dT)-Schwanzes kovalent mit primären Aminen in der Nylonmembran verknüpft.

In einem weiteren Aspekt betrifft die Erfindung außerdem einen Festphasen-DNA-Microarray zur gleichzeitigen Amplifikation von Nucleinsäuren in flüssiger und fester Phase, auf dem an x räumlich beabstandeten Position x innere PCR-Primer P₃1, P₃2, P₃3 ... P3x irreversibel an die Festphase gebunden sind und auf dem weiterhin alle für die Durchführung des Verfahrens erforderlichen Komponenten umfassen die DNA-Polyermerase, geeignete Puffersubstanzen, sämtliche Desoxynucleosidtriphosphate und die x äußeren PCR-Primer P₁1, P₂1, P,2, P₂2, P₂3,... P₁x, P₂x.

Es können beliebige, beispielsweise handelsübliche, PCR-Reagenzien verwendet werden. Die Aufbringung auf den festen Träger erfolgt nach herkömmlichen Verfahren, beispielsweise durch Aufpipettieren. Anschließend wird dehydratisiert, beispielsweise durch Trocknen an der Luft, im vakuum oder im Trockenschrank, über einem üblichen Trocknungsmittel in einen Exsikkator oder durch Lyophilisation (Gefriertrocknung). Die Lyophilisation ist bevorzugt, da es sich um ein besonders schnelles und schonendes Dehydratisierungsverfahren handelt. Die Endkonzentrationen können einfach eingestellt werden, indem die PCR-Reagenzien in so konzentrierterer Form aufgebracht werden, daß sich nach der Rehydratisierung in einem bestimmten Volumen Flüssigkeit die gewünschten Reaktionsparameter wie insbesondere Konzentrationen und pH-Wert ergeben.

Die Aufbringung kann auch räumlich getrennt erfolgen.

Die PCR-Reagenzien sind auf dem festen Träger über lange Zeit, z.B. mindestens 6 Monate, ohne Kühlung stabil.

Die inneren Primer sind bereits im voraus (d.h. vor der Aufbringung und Dehydratisierung der PCR-Reagenzien) kovalent auf der Oberfläche des festen Trägers gebunden bzw. immobilisiert.

Im Gebrauch werden die in der zu analysierenden Probe gegebenenfalls vorhandenen Proben-Nucleinsäuren in wäßriger Lösung zugegeben, wodurch die PCR-Reagenzien rehydrieren und die Amplifikation im Falle des Vorhandenseins einer vom Design des analytischen Ansatzes antizipierten "Template"-Gegensequenz auf dem festen Träger abläuft, ohne daß die Zugabe weiterer Reagenzien notwendig ist.

Die Analyse/Detektion erfolgt nach einer bevorzugten Ausführungsform mit Hilfe von kovalent an dem Festphasen-DNA-Microarray als Extensionsprodukte gebildeten und markierten DNA-Sequenzen und durch Bestimmung der Lokalisierung der Markierung auf der Oberfläche wie beispielsweise aus EP 0 373 203 bekannt.

Beispielsweise kann ein Fluorochrom als Marker in das Amplikon eingebaut werden, dessen Fluoreszenzemission dann detektiert oder gemessen wird. Da der Einbau der Markierung ortsgebunden erfolgt, können die Markierungsreaktionen, gewünschtenfalls auch in Echtzeit, nachgewiesen bzw. beobachtet werden.

Die Markierungs- und Detektionstechniken unterliegen keinen besonderen Beschränkungen. Beispielsweise können radioaktiv oder fluoreszenzmarkierte Nucleotide verwendet werden. Die Fluoreszenzmarkierung hat dabei den Vorteil, daß keine besonderen Schutzvorkehrungen wie beim Arbeiten mit radioaktivem Material getroffen werden müssen. Der Nachweis der Hybridisierung kann beispielsweise durch konfokale Laser-Scanning-Mikroskopie erfolgen.

Eine weitere Möglichkeit ist die Verwendung von digoxigeninmarkierten spezifischen Sonden (Oligonucleotiden). Der Nachweis der Hybridisierung kann hier beispielsweise durch eine sich anschließende Antikörper- und Farbreaktion erfolgen, z.B. mit Hilfe eines poly- oder monoklonalen Digoxigenin-Antikörpers, der z.B. mit alkalischer Phosphatase konjugiert ist. Die alkalische Phosphatase setzt beispielsweise Nitroblautetrazoliumchlorid (NBT) oder 5-Brom-4-chlor-3-indolylphosphat (BCIP) unter Bildung eines blauen Farbstoffs um.

Der zur Ausführung eines der erfindungsgemäßen Verfahren vorgeschlagene Festphasen-DNA-Microarray liegt in weiterer Ausgestaltung in Form eines sog. "Lab-on-a-Chip"-Systems vor, bei dem alle notwendigen Einrichtungen zum Mischen der Reaktionsteilnehmer sowie zur Temperaturregelung, ggf. einschließlich einer Steuereinheit für einzelne oder alle Einrichtungen, auf dem Microarray integriert sind. Ein solches, für den erfindungsgemäßen Einsatz grundsätzlich geeignetes System wird beispielsweise von Köhler et al. in transcript Laborwelt, Nr. 2 (2000), S. 5 ff. "Chipthermocycler" beschrieben und kann vom Fachmann in Kenntnis der vorliegenden Erfindung ohne zumutbaren Aufwand leicht konfiguriert und betrieben werden. Alternativ kann die Steuerung der erforderlichen Verfahrensparameter vollständig oder teilweise extern wie z.B. in einer Rechneranlage erfolgen.

Kurz zusammengefaßt vereinen das allgemeine erfindungsgemäße Verfahren zur spezifischen Bestimmung von DNA-Sequenzen mittels paralleler Amplifikation durch verschachtelte PCR in einem kombinierten Flüssigphasen/Festphasen-DNA-Microarraysystem sowie die für bestimmte Anwendungsgebiete modifizierten Ableitungen desselben in sich die Sensitivität der PCR, die Parallelität der Mikroarrayanalytik und die Spezifität des Systems Primer/"Template"/Polymerase mit dem Vorteil der stabilen dehydrierten Bindung von Reagenzien auf einem Microarray.

Die vorliegende Erfindung wird anhand der folgenden Beispiele unter Bezugnahme auf die anhängenden Figuren nachfolgend näher erläutert. Es wird jedoch ausdrücklich darauf hingewiesen, dass die Beispiele lediglich der Veranschaulichung einzelner bestimmter Ausführungsformen der vorliegenden Erfindung dienen, nicht aber als Beschränkung des allgemeinen erfinderischen Gedankens aufgefasst werden soll. Vom Fachmann können zahlreiche Modifikationen und Optimierungen der konkreten Ausführungsbeispiele vorgenommen werden, ohne den Schutzbereich der vorliegenden Erfindung, der allein durch die anhängenden Ansprüche festgelegt wird, zu verlassen.

### Beispiel 1

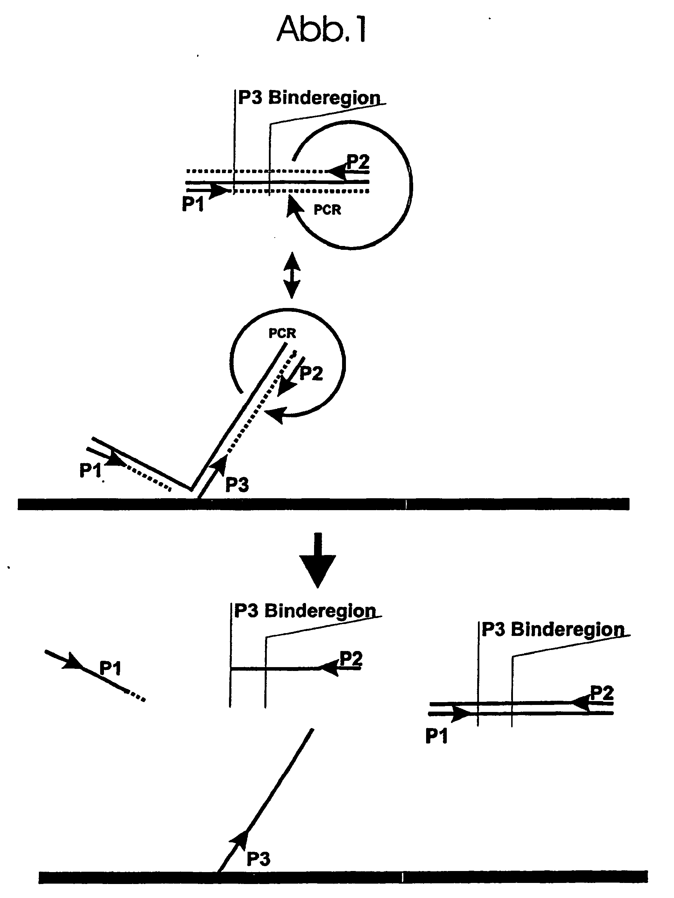

Abb.1: Spezifischer Nachweis einer bekannten Sequenz. Die obere Hälfte der Abb. schematisiert die Vorgänge während der Amplifikation, die untere Hälfte die Situation zum Zeitpunkt der Detektion. Es sind mindestens 1 Lösungsprimerpaar (P₁ und P₂) und mindestens 1 Festphasenprimer (P₃) vorhanden. P₃ hat seine Binderegion innerhalb des Amplikons, welches von P₁ und P₂ amplifiziert wird. Da nach jedem Zyklus bei 90 - 95°C denaturiert wird, was extremen Waschschritten (strippen) entspricht, bleiben nach Ende der Reaktion nur die kovalent gebundenen Reaktionsprodukte an der Festphase zurück (vgl. Abb. 1 unten). Alle anderen Reaktionsprodukte befinden sich im Überstand und bleiben bei der Detektion unberücksichtigt.

Das vorliegende Beispiel betrifft den spezifischen Nachweis der Anwesenheit einer bekannten, für Salmonellen charakteristischen Sequenz in einer zu analysierenden Lebensmittelprobe. Die Aufbereitung des Probenmaterials erfolgt in Übereinstimmung mit der DIN-Vorschrift 10135, welche speziell den Nachweis von Salmonellen betrifft.

Die in 100 µl wäßriger Lösung aufgenommene Proben-DNA wird auf einen Microarray aus aktiviertem Glas, auf dem sich bereits sämtliche übrigen Reaktionsteilnehmer in lyophilisierter Form befinden, wobei alle Komponenten mit Ausnahme der kovalent an der festen Phase gebundenen P₃-Primer reversibel immobilisiert sind, aufpipettiert.

Durch Zugabe der Probenlösung werden die lyophilisierten Komponenten rehydratisiert und stehen dem folgenden Reaktionsablauf mit folgenden Konzentrationen zur Verfügung. Die Primer P₁ und P₂ liegen in Konzentrationen von 100 nM bzw. 600 nM vor, während der Primer P₃ eine Konzentration von 50 pM aufweist. In der wäßrigen Probenlösung mit einem pH-Wert von 8,3 befinden sich die üblichen PCR-Standardpufferkomponenten 50 mM KCl, 10 mM Tris/HCl, 1,5 mM MgCl₂, sowie die drei Nucleotide dATP, dCTP und dGTP in einer jeweiligen Konzentration von 200 µM. Die dTTP-Konzentration des Systems beträgt 75 µM, diejenige an Biotin-dUTP beträgt 50 µM. Weiterhin umfasst das System 6,4 Einheiten (units) Taq-Polymerase (AmpliTaq™ - Gold, Perkin-Elmer).

Die Reaktion wird durch eine 10 Minuten lange Aktivierung der Polymerase gestartet und läuft bei einer jeweiligen Annealingtemperatur von 65°C und einer jeweiligen Denaturierungstemperatur von 94°C über 50 Zyklen, wobei ein jeder Zyklus unter Verwendung eines Thermcyclers PTC 200 (MJ Research) die folgenden Inkubationszeiten umfasst: 45 Sekunden 94°C, 60 Sekunden 65°C, 120 Sekunden bei 72°C.

Das in die kovalent gebundenen Produkte eingebaute Biotin wird nach der Reaktion mittels Streptavidin-Fluorochrom-Konjugat in an sich bekannter Weise angefärbt und einer Detektion zugeführt.

### Beispiel 2

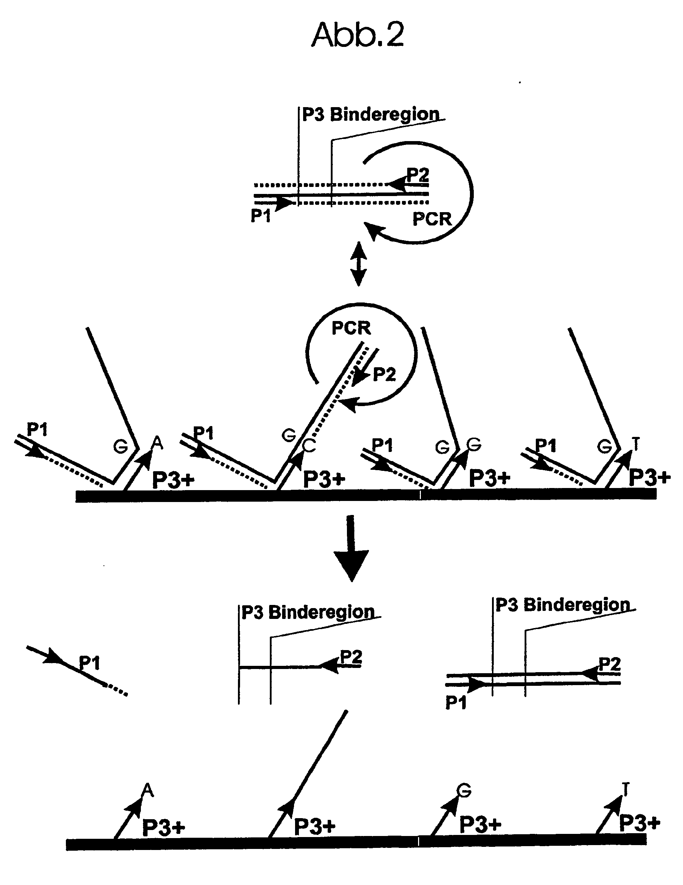

Abb.2: Allotypbestimmung: Spezifische Bestimmung der Identität eines 3'-ständigen Nucleotids mittels Primerfamilien. Die obere Hälfte der Abb. schematisiert die Vorgänge während der Amplifikation, die untere Hälfte die Situation zum Zeitpunkt der Detektion.

Unter den in Beispiel 1 genannten Reaktionsbedingungen wird eine Konsensus-PCR (vgl. z.B. P. Wordsworth, "Techniques used to define human MHC antigens: polymerase chain reaction and oligonucleotide probes", *Immunol Lett,* 1991 Jul ; 29 (1-2) :37-9) durchgeführt, bei der alle 300 Varianten vom insgesamt 270 Nucleotide umfassenden Exon II des DR-Gens (HLA-Klasse II) amplifiziert werden. Die intern bindenden Festphasenprimer P3 besitzen Längen von 18 bis 27 Nucleotide und sind Mitglieder von insgesamt 250 Familien, deren Anzahl benötigt wird, um über Redundanzen eine eindeutige Identifizierung eines Genotyps auch bei Heterozygotie zu ermöglichen, wobei sich jedes einzelne Mitglied der Primerfamilie hinsichtlich des Nukleotids an seinem 3'-Ende von jedem anderen Mitglied unterscheidet (vgl. Abb. 2).

Aufgrund der im wesentlichen gleichen Länge der vorhandenen Primer beträgt die Annealingtemperatur der Amplifikationsreaktion 65°C, so daß die gewünschten Extensionsprodukte unter den gewählten Bedingungen bevorzugt nur an denjenigen definierten Positionen des DNA-Microarrays gebildet werden, welche durch die Anwesenheit des zu der zu bestimmenden Variantensequenz im wesentlichen exakt komplementären Primers gekennzeichnet sind.

Im Anschluss an die Detektion gemäß Beispiel 1 wird das erhaltene Fluoreszenzmuster mit den anhand der Einzelsequenzen der verschiedenen Allele zu erwartenden Signalmusterpermutationen verglichen. Die jeweils durch den Vergleich aufgefundenen Übereinstimmungen ermöglichen die zweifelsfreie Identifizierung der zu untersuchenden Genotyp-Varianten.

### Beispiel 3

Abb.3: "Sequence Tag Aquisition": Spezifische Signatur-Sequenzierung ausgehend von einer bekannten Primersequenz mittels Primerfamilien. Die obere Hälfte der Abb. schematisiert die Vorgänge während der Amplifikation, die untere Hälfte die Situation zum Zeitpunkt der Detektion. In der oberen Hälfte sind die Nebenreaktionen an der Festphase im Gegensatz zu Abb. 1 und 2 aus übersichtgründen nicht dargestellt.

Wie bei Beispiel 2 wird eine Konsensus-PCR durchgeführt. In diesem Fall wird die 16S-rDNA eines unbekannten Bakteriums amplifiziert. Das Amplikon ist in diesem Fall ca. 1200 bp lang. Sequenziert werden soll jedoch lediglich ein interner Bereich von 6 Nuklotiden in einem variablen, d.h. nicht konservierten Bereich. Dieser unbekannte Sequenzabschnitt wird ausgehend von einer konservierten Region ermittelt. Hierzu werden Festphasenprimerfamilien verwendet, die sich, ausgehend von einer konservierten Region, am 3'-Ende jeweils durch die letzte Base unterscheiden, so daß sich insgesamt 4⁶ entsprechend 4096 Varianten ergeben (vgl. Abb. 3). Die Annealingtemperatur und die Gesamtlänge der Primer sind an die gewünschte Spezifität angepaßt (s. Beispiel 2). Wie in Beispiel 2 angegeben, weisen die Primer Längen zwischen 18 und 27 Nukleotiden auf.

Die Detektion erfolgt wie zuvor in Beispiel 2 beschrieben.

Die Auswertung der erhaltenen Daten erfolgt auf der Grundlage des Microarray-Designs. Durch die vom Design vorgegebene Gruppierung der einzelnen Reaktionsbereiche in Felder, die mit der ersten, zweiten, dritten, vierten, fünften und sechsten Position der zu bestimmenden Sequenz korrespondieren, erfolgt die Bestimmung des 6 Nukleotide umfassenden DNA-Teilbereichs durch sukzessives Ablesen der für jedes Feld erhaltenen Daten. Mit anderen Worten wird zuerst das Feld ausgewertet, in welchem ausgehend vom Design des Arrays die erste zu bestimmende Base ermittelt wird, bevor in Kenntnis des Ergebnisses aus diesem ersten Feld die Bestimmung der Base an der zweiten Position der zu ermittelnden Sequenz vorgenommen wird, u.s.w. Die jeweilige Untersuchung eines Feldes erfolgt durch Erfassung und Zuordnung der größten gemessenen Signalstärke. Selbstverständlich können die Feldmessungen auch parallelisiert ablaufen. Ergänzend wird in diesem Zusammenhang auf Abb. 3 verwiesen.

### Beispiel 4

Abb.4: Signatur-Sequenzierung ausgehend von einer unbekannten Sequenz mittels Primer-Hexameren. Die obere Hälfte der Abb. schematisiert die Vorgänge während der Amplifikation, die untere Hälfte die Situation zum Zeitpunkt der Detektion. In der oberen Hälfte sind die Nebenreaktionen an der Festphase im Gegensatz zu Abb. 1 und 2 aus Übersichtgründen nicht dargestellt.

Eine unbekannte Sequenz wird mittels spezifischer Primer nach inverser PCR amplifiziert. Die inverse PCR ist im Stand der Technik bekannt (vgl. z.B. C. R. Newton, A. Graham: "PCR", Spektrum Akademischer Verlag Heidelberg Berlin Oxford 1994, Seite 120ff. und die dort angegebenen weiteren Literaturstellen). In diesem Fall wird die "Border"-Sequenz eines integrierten Transgenkonstruktes im Mais-Kerngenom bestimmt. Das Amplikon umfaßt 500 bp. Die Festphasenprimer P₃ sind alle 6 Nucleotide lang und unterscheiden sich in ihrer Sequenz. Es sind 4096 unterschiedliche Festphasenprimervarianten auf dem Chip. Es werden 1000 Kopien der Mais-DNA eingesetzt. Wiederum werden Standard-PCR-Bedingungen angewandt (s.o.). Allerdings wird die Annealingtemperatur nach Zyklus 20 auf 30°C erniedrigt, um die Festphasen-Amplifikation mit den Hexameren zu begünstigen.

Die Bestimmung der Sequenz erfolgt anschließend durch Ermittlung der spezifisch gebildeten Extensionsprodukte, Zuordnung dieser ermittelten Werte zu den definierten Positionen des DNA-Microarrays, und Zusammenstellung der zu bestimmenden Sequenz im Rahmen einer ggf. Computer-unterstützten Kombinatorik, wobei die tatsächliche Aneinanderreihung der Einzeldaten zur Gesamtsequenz unter Nutzung der jeweils überlappenden Sequenzbereiche vorgenommen wird.

## Patentansprüche

1. Verfahren zur spezifischen Bestimmung von DNA-Sequenzen mittels Amplifikation in einem kombinierten Flüssig-phasen/Festphasen-DNA-Microarraysystem, indem man eine verschachtelte Polymerasekettenreaktion zur gleichzeitigen Amplifikation von Nukleinsäuren in der festen und in der flüssigen Phase unter Verwendung von x PCR-Primersätzen aus jeweils mindestens 3 PCR-Primern P₂1, P₂1, P₃1, P₁2, P₂2, P₃2, P₁3, P₂3, P₃3, ... P₁x, P₂x, P₃x durchführt, wobei x eine natürliche Zahl bedeutet und der Anzahl der zu bestimmenden DNA-Sequenzen entspricht, und wobei für jeden der x PCR-Primersätze
(a) zwei äußere PCR-Primer P₁, P₂ in der flüssigen Phase vorliegen und so ausgewählt werden, daß sie an stromaufwärts und stromabwärts der zu amplifizierenden Ziel-DNA-Sequenz liegende DNA-Teilsequenzen hybridisieren,
(b) ein innerer PCR Festphasenprimer P₃ so ausgewählt wird, daß er an eine innerhalb der zu bestimmenden Ziel-DNA-Sequenz liegende DNA-Teilsequenz hybridisiert und in der Lage ist, ein P₃-Extensionsprodukt zu bilden, und
(c) die x äußeren PCR Primer P₁1, P₂1, P₁2, P₂2, P₁3, P₂3, ... P₁x, P₂x in der flüssigen Phase gegenüber den x inneren PCR-Primern P₃1, P₃2, P₃3, ... P₃x im Überschuss vorliegen, und
(d) die x inneren PCR-Primer P₃1, P₃2, P₃3, ... P₃x an x räumlich beabstandeten Positionen unter Bildung eines DNA-Microarrays irreversibel an eine Festphase gebunden vorliegen, und
(e) man die Bestimmung durch Ermittlung des Vorhandenseins eines P₃-Extensionsproduktes an der Position des DNA-Microarrays durchführt.

2. Verfahren nach Anspruch 1 zur Bestimmung von Punktmuta-tionen, bei dem durch die Polymerasekettenreaktion sämtliche x zu bestimmenden Varianten amplifiziert werden, wobei
(a) P₃x Primerfamilien verwendet werden, innerhalb derer sich jedes einzelne Mitglied P₃x-N einer Familie hinsichtlich des Nukleotids an seinem 3'-Ende von jedem anderen Mitglied derselben Familie unterscheidet, wobei N die unterschiedlichen Nukleotide A, C, G und T bezeichnet, und die Annealingtemperatur der Amplifikationsreaktion so ausgewählt wird, daß die gewünschten Extensionsprodukte nur an denjenigen Positionen des DNA-Microarrays gebildet werden, welche durch die Anwesenheit des zu der zu bestimmenden Punktmutation im wesentlichen exakt komplementären Primers gekennzeichnet sind, und
(b) zur Amplifikation eine DNA-Polymerase ohne 3'→5'-Exonuclease-Aktivität verwendet wird.

3. Verfahren nach Anspruch 1 zur Bestimmung der Sequenz von DNA-Teilbereichen, bei dem durch die Polymerasekettenreaktion sämtliche x zu bestimmenden Sequenzen amplifiziert werden, wobei
(a) P₃x Primerfamilien verwendet werden, innerhalb derer sich jedes einzelne Mitglied P₃x-N,... P₃x-Nn einer Familie hinsichtlich seiner 1 bis n Nukleotide am 3'-Ende von jedem anderen Mitglied derselben Familie unterscheidet, wobei N die unterschiedlichen Nukleotide A, C, G und T bezeichnet, und n die Länge der zu bestimmenden Sequenz(en) angibt, und die Annealingtemperatur der Amplifikationsreaktion so ausgewählt wird, daß die gewünschten Extensionsprodukte nur an denjenigen Positionen des DNA-Microarrays gebildet werden, welche durch die Anwesenheit des zu der zu bestimmenden Sequenz im wesentlichen exakt komplementären Primers gekennzeichnet sind, und
(b) zur Amplifikation eine DNA-Polymerase ohne 3'→5'-Exonuclease-Aktivität verwendet wird.

4. Verfahren nach Anspruch 1 zur Bestimmung der Sequenz von unbekannten DNA-Teilbereichen, bei dem durch die Polymerasekettenreaktion die Abfolge sämtlicher n Nukleotide der zu bestimmenden Sequenzen ermittelt wird, wobei
(a) ein sämtliche Permutationen umfassender Satz von inneren Primern P₃ mit einer bestimmten Länge n verwendet wird, wobei mehrere Primer mit einer jeweils unterschiedlichen Region der zu bestimmenden Sequenz exakt hybridisieren und Extensionsprodukte definierter Länge bilden können, und sich jeder einzelne dieser Primer an einer anderen Position des DNA-Microarrays befindet,
(b) zur Amplifikation eine DNA-Polymerase ohne 3'→5'-Exonuclease-Aktivität verwendet wird, und
(c) die Bestimmung der Sequenz erfolgt durch Ermittlung der in Schritt (a) gebildeten Extensionsprodukte, Zuordnung dieser ermittelten Werte zu den Positionen des DNA-Microarrays, und Zusammenstellung der zu bestimmenden Sequenz im Rahmen einer ggf. Computer-unterstützten Kombinatorik, wobei die tatsächliche Aneinanderreihung der Einzeldaten zur Gesamtsequenz unter Nutzung der jeweils überlappenden Sequenzbereiche vorgenommen wird.

5. Verfahren nach einem der vorstehenden Ansprüche, wobei die Schmelztemperaturen der zwei äußeren PCR-Primer P₁ und P₂ gegenüber derjenigen des inneren Primers P₃ unterschiedlich sind.

6. Verfahren nach einem der vorstehenden Ansprüche, wobei die Festphase unter Metalloberflächen, mit SiO₂ bedampften Metalloberflächen, Metall/Halbmetalloxidoberflächen, Glasoberflächen, Polymeroberflächen, Nylonmembranen oder Nitrocellulosemembranen ausgewählt ist.

7. Verfahren nach Anspruch 6, wobei die Metalloberfläche aus Aluminium oder Gold, die Metall/Halbmetalloxidoberfläche aus Al₂O₃ oder SiO₂, die Glasoberfläche aus Quarzglas und die Polymeroberfläche aus Polypropylen, Polymethylmethacrylat oder Cycloolefin-Copolymeren ist.

8. Verfahren nach Anspruch 7, wobei die x inneren PCR-Primer P₃1, P₃2, P₃3, ... P₃x an eine Metall/Halbmetalloxidoberfläche oder Glasoberfläche über ein bifunktionelles Silan, das am Siliciumatom ein(e), zwei oder drei hydrolysierbare(s) Atom(e) oder Gruppe(n) aufweist, gebunden sind.

9. Festphasen-DNA-Microarray zur gleichzeitigen Amplifikation von Nukleinsäuren in flüssiger und fester Phase mittels verschachtelter PCR, umfassend einen festen Träger, auf dessen Oberfläche an x räumlich beabstandeten Positionen x innere PCR-Primer P₃1, P₃2, P₃3, ... P₃x irreversibel an die Festphase gebunden sind und auf dem weiterhin alle für die Durchführung des Verfahrens erforderlichen Komponenten reversibel dehydratisiert auf der Oberfläche vorliegen, wobei die Komponenten umfassen die DNA-Polymerase, geeignete Puffersubstanzen, sämtliche Desoxynukleosidtriphosphate und die x äußeren PCR-Primer P₁1, P₂1, P₁2, P₂2, P₁3, P₂3, ... P₁x, P₂x, wobei x eine natürliche Zahl bedeutet und der Anzahl der zu amplifizierenden DNA-Sequenzen entspricht.

10. Festphasen-DNA-Microarray nach Anspruch 9, bei dem mindestens eine Einrichtung zum Heizen, Kühlen, Temperaturregeln, Mischen, Messen und/oder Steuern integriert ist.

## Claims

1. Method for the specific determination of DNA sequences by means of parallel amplification in a combined liquid-phase/solid-phase-DNA-microarray system by performing a nested polymerase chain reaction using x PCR primer sets each of at least 3 PCR primers P₁1, P₂1, P₃1, P₁2, P₂2, P₃2, P₁3, P₂3, P₃3, ... P₁x, P₂x, P₃x, whereby x signifies a natural number (positive integer) and corresponds to the number of DNA sequences to be determined, and whereby for each of the x PCR primer sets
(a) two outer PCR primers P₁, P₂ are present in the liquid phase and are chosen in such a way that they hybridise onto DNA sub-sequences lying upstream and downstream of the target DNA sequence that is to be amplified,
(b) one inner PCR primer P₃ is chosen in such a way that it hybridises onto a DNA subsequence lying within the target DNA sequence that is to be determined, and is able to form a P₃ extension product, and
(c) the x outer PCR primers P₁1, P₂1, P₁2, P₂2, P₁3, P₂3, ... P₁x, P₂x are present in the liquid phase in excess relative to the x inner PCR primers P₃1, P₃2, P₃3, ... P₃x, and
(d) the x inner PCR primers P₃1, P₃2, P₃3, ... P₃x are present irreversibly bonded to a solid phase at x spatially separated defined positions, forming a DNA microarray, and
(e) the determination is performed by ascertaining the presence of a P₃ extension product at the defined position of the DNA microarray.

2. Method of claim 1 for determining point mutations by means of parallel amplification, in which a polymerase chain reaction, through which all of the x variants to be determined are amplified, is carried out, whereby
(a) P₃x primer families are used, wherein each individual member P₃x-N of the primer family differs from each other member with regard to the nucleotide at its 3' terminus, wherein N describes the different nucleotides A, C, G and T, and the annealing temperature of the amplification reaction is chosen in such a way that the desired extension products are formed at only those defined positions of the DNA microarray that are **characterized by** the presence of the primer that is essentially exactly complementary to the point mutation to be determined, and
(b) a DNA polymerase without any 3'→5' exonuclease activity is used for amplification.

3. Method of claim 1 for the determination of the sequence of DNA sub-regions by means of parallel amplification in which a polymerase chain reaction, through which all of the x sequences to be determined are amplified, is carried out, whereby
(a) at least P₃x primer families are used, wherein each individual member P₃x-N, ... P₃x-Nn of the primer family differs from each other member with regard to its 1 to n nucleotides at the 3' terminus, wherein N describes the different nucleotides A, C, G and T, and n indicates the length of the sequence(s) to be determined, and the annealing temperature of the amplification reaction is chosen in such a way that the desired extension products are formed at only those defined positions of the DNA microarray that are **characterized by** the presence of the primer that is essentially exactly complementary to the sequence that is to be determined, and
(b) a DNA polymerase without any 3'→5' exonuclease activity is used for amplification.

4. Method of claim 1 to determine the sequence of unknown DNA sub-regions by means of parallel amplification in which a polymerase chain reaction is carried out through which the sequence of all of the n nucleotides of the sequences to be determined is ascertained, whereby
(a) a set of inner primers P₃ with a defined length n and encompassing all of the permutations is used, whereby several primers can hybridise exactly with and can form extension products of a defined length with in each case a different region of the sequence to be determined, and each individual one of these primers is situated at a different defined position of the DNA microarray,
(b) a DNA polymerase without any 3'→5' exonuclease activity is used for amplification, and
(c) the determination of the sequence takes place by ascertaining the extension products formed in Step (a), assigning these determined values to the defined positions of the DNA microarray, and compilation of the sequence to be determined in the context of a combinatorial analysis, computer-assisted if necessary, whereby the actual alignment of the individual data to the overall sequence is performed using the respective overlapping sequence regions.

5. Method according to one of the foregoing Claims, whereby the melting temperatures of the two outer PCR primers P₁ and P₂ are different to that of the inner primer P₃.

6. Method according to one of the foregoing Claims, wherein the solid phase is chosen from among metal surfaces, metal surfaces vapour-deposited with SiO₂, metal/semimetal oxide surfaces, glass surfaces, polymer surfaces, nylon membranes or nitrocellulose membranes.

7. Method according to Claim 6, wherein the metal surface is made of aluminium or gold, the metal/semimetal oxide surface is made of Al₂O₃ or SiO₂, the glass surface is made of quartz glass and the polymer surface is made of polypropylene, polymethylmethacrylate or cycloolefine copolymers.

8. Method according to Claim 7, wherein the x inner PCR primers P₃1, P₃2, P₃3, ... P₃x are bonded to a metal/semimetal oxide surface or glass surface via a bifunctional silane that has one, two or three hydrolysable atom(s) or group(s) on the silicon atom.

9. Solid-phase-DNA-microarray for simultaneous amplification of nucleic acids in liquid and on solid phase using a nested PCR reaction, comprising a solid support onto which x inner PCR primers P₃1, P₃2, P₃3, ... P₃x are irreversibly bonded to the solid phase at x spatially separated positions, and on which in addition all of the components needed for the reaction(s) to be carried out, are present reversibly dehydrated on the surface, which components include said DNA polymerase, suitable buffer substances, all of the deoxynucleoside triphosphates and the x outer PCR primers P₁1, P₂1, P₁2, P₂2, P₁3, P₂3, ... P₁x, P₂x, wherein x is a natural number.

10. Solid-phase-DNA-microarray according to Claim 9 wherein at least one of a device for heating, cooling, temperature control, mixing, measurement and control are integrated.

## Revendications

1. Procédé de détermination spécifique de séquences d'ADN par amplification dans un système combiné de microréseau (microarray) d'ADN en phases liquides/phases solides, dans lequel on réalise une réaction imbriquée en chaîne par polymérase (PCR) pour l'amplification simultanée d'acides nucléiques dans la phase solide et liquide en utilisant x séquences d'amorce PCR constituées respectivement d'au moins 3 amorces PCR P₁1, P₂1, P₃1, P₁2, P₂2, P₃2, P₁3, P₂3, P₃3, ... P₁x, P₂x, P₃x, dans lesquelles x représente un nombre naturel et correspond au nombre de séquences d'ADN à déterminer, et dans lesquelles pour chacune des x séquences d'amorce PCR
(a) deux amorces PCR externes P₁, P₂ se trouvent dans la phase liquide et sont choisies de manière à ce qu'elles s'hybrident sur des sous-séquences d'ADN situées en amont et en aval de la séquence d'ADN cible à amplifier,
(b) une amorce PCR interne en phase solide P₃ est choisie de manière à ce qu'elle s'hybride sur une sous-séquence d'ADN située à l'intérieur de la séquence cible d'ADN à déterminer et soit en mesure de former un produit d'extension P₃, et
(c) les x amorces PCR externes P₁1, P₂1, P₁2, P₁3, P₂3, ...P₁x, P₂x dans la phase liquide sont présentes en excès par rapport aux x amorces PCR internes P₃1, P₃2, P₃3, ... P₃x, et
(d) les x amorces PCR internes P₃1, P₃2, P₃3, ... P₃x sont présentes irréversiblement liées à une phase solide à x localisations écartées spatialement en formant un microréseau d'ADN (DNA-microarray), et
(e) on effectue la détermination par identification de la présence d'un produit d'extension P₃ à la localisation définie du microréseau d'ADN.

2. Procédé selon la revendication 1 pour la détermination de mutations ponctuelles, dans lequel on amplifie l'ensemble des x variantes à déterminer par réaction en chaîne par polymérase, dans lequel
(a) on utilise les familles d'amorces P₃x, à l'intérieur desquelles chaque membre particulier P₃x-N d'une famille diffère de tout autre membre de la même famille en ce qui concerne le nucléotide à son extrémité 3', N désignant les différents nucléotides A, C, G et T, et on choisit la température d'annelage de la réaction d'amplification de manière à ce que les produits d'extension souhaités soient formés seulement aux localisations du microréseau d'ADN qui sont **caractérisées par** la présence de l'amorce qui est essentiellement exactement complémentaire de la mutation ponctuelle à déterminer, et
(b) on utilise pour l'amplification une ADN-polymérase sans activité 3'→5' exonucléase.

3. Procédé selon la revendication 1 pour la détermination de la séquence de sous-régions d'ADN, dans lequel on amplifie par la réaction en chaîne par polymérase l'ensemble des x séquences à déterminer, dans lequel
(a) on utilise les familles d'amorces P₃x, à l'intérieur desquelles chaque membre particulier P₃x-N, ... P₃x-Nn d'une famille diffère de tout autre membre de la même famille en ce qui concerne ses nucléotides 1 à n à l'extrémité 3', N désignant les différents nucléotides A, C, G et T, et n indique la longueur de la (des) séquence(s) à déterminer, et on choisit la température d'annelage de la réaction d'amplification de manière à ce que les produits d'extension souhaités soient formés seulement aux localisations du microréseau d'ADN qui sont **caractérisées par** la présence de l'amorce qui est essentiellement exactement complémentaire de la séquence à déterminer, et
(b) on utilise pour l'amplification une ADN-polymérase sans activité 3'→5' exonucléase.

4. Procédé selon la revendication 1 pour la détermination de la séquence de sous-régions d'ADN inconnues, dans lequel on identifie la suite d'un l'ensemble de n nucléotides de la séquence à déterminer par la réaction en chaîne par polymérase, dans lequel
(a) on utilise un jeu d'amorces internes P₃ avec une longueur n déterminée comprenant l'ensemble des permutations, plusieurs amorces pouvant s'hybrider exactement avec une région respectivement différente de la séquence à déterminer et former des produits d'extension de longueur définie, et chacune de ces amorces individuelles se trouve à une localisation différente du microréseau d'ADN,
(b) on utilise pour l'amplification une ADN-polymérase sans activité 3'→5' exonucléase, et
(c) on effectue la détermination de la séquence par identification des produits d'extension formés à l'étape (a), attribution de ces valeurs identifiées aux localisations définies du microréseau d'ADN, et compilation de la séquence à déterminer dans le cadre d'une analyse combinatoire éventuellement assistée par ordinateur, par lequel on réalise l'alignement effectif des données individuelles pour obtenir la séquence globale en utilisant les régions de recouvrement de séquences.

5. Procédé selon l'une des revendications précédentes, dans lequel les températures de fusion des deux amorces PCR externes P₁ et P₂ sont différentes de celle de l'amorce interne P₃.

6. Procédé selon l'une des revendications précédentes, dans lequel on choisit la phase solide parmi les surfaces de métal, les surfaces de métal revêtues de SiO₂ par évaporation, les surfaces d'oxyde de métal/métalloïde, les surfaces de verre, les surfaces de polymère, les membranes de Nylon ou les membranes de nitrocellulose.

7. Procédé selon la revendication 6, dans lequel la surface de métal est en aluminium ou en or, la surface d'oxyde de métal/métalloïde en Al₂O₃ ou SiO₂, la surface de verre en verre de silice et la surface de polymère en polypropylène, polyméthacrylate de méthyle ou copolymères de cyclooléfines.

8. Procédé selon la revendication 7, dans lequel les x amorces PCR internes P₃1, P₃2, P₃3 ... P₃x sont liées à une surface d'oxyde de métal/métalloïde ou une surface de verre via un silane bifonctionnel qui présente sur l'atome de silicium un, deux ou trois atomes ou groupes hydrolysables.

9. Microréseau d'ADN en phases solides pour l'amplification simultanée d'acides nucléiques en phase liquide et solide par PCR imbriquée, comprenant un support solide, sur la surface duquel x amorces PCR internes P₃1, P₃2, P₃3, P₂3, ... P₃x, sont liées irréversiblement à la phase solide à x localisations écartées spatialement et sur lequel en outre tous les composants nécessaires pour la réalisation du procédé sont présent sur la surface dans un état réversiblement déshydraté, les composants comprenant l'ADN polymérase, des substances tampons appropriées, tous les désoxynucléoside-triphosphates et les x amorces PCR externes P₁1, P₂1, P₁2, P₂2, P₁3, P₂3, ... P₁x, P₂x, dans lesquelles x représente un nombre naturel et correspond au nombre de séquences d'ADN à amplifier.

10. Microréseau d'ADN en phases solides selon la revendication 9, dans lequel est intégré au moins un dispositif pour le chauffage, le refroidissement, la régulation de température, le mélange, la mesure et/ou la régulation.
